# EUROPEAN PATENT APPLICATION

(11) **EP 1 876 237 A1**
(43) Date of publication of application: **09.01.2008**
(21) Application number: 06731708.1
(22) Date of filing: 12.04.2006
(51) Int. Cl.: C12N 15/09, A01K 67/02, A61K 45/00, A61P 15/00, A61P 15/08

(54) **PROMOTER FOR INTRODUCING EXTRACELLULAR SUBSTANCE INTO MAMMALIAN OVUM AND INTRODUCTION METHOD**

(30) Priority: 27.04.2005 JP 2005129198
(71) Applicant: Japan Science and Technology Agency, Kawaguchi-shi, Saitama 332-0012 (JP)
(72) Inventor: MIYADO, Kenji, 1570074 (JP); MIYADO, Mami, 1570074 (JP); AKUTSU, Hidenori, 1570074 (JP)
(74) Representative: Swinkels, Bart Willem
(86) International application number: PCT/JP2006/307772
(87) International publication number: WO 2006/117991

(57) **Abstract**

The present invention provides a method, upon introducing an extracellular substance, such as sperm, a cell nucleus, a nucleic acid, and a protein, into a mammalian oocyte, for introducing the extracellular substance into a mammalian oocyte, which is safe for the mammalian oocyte, enables efficient introduction, and is technically easy. By treating the mammalian oocyte with a tubulin-polymerization inhibitor such as vinblastine, membrane fusion between the oocyte and the sperm is promoted, and the sperm and the oocyte are fused without making a hole in the oolemma and fertilization can be achieved. The method of the present invention can be applied not only to the case of introducing sperm into a mammalian oocyte, but also to the case of introducing an extracellular substance, such as a cell nucleus, a nucleic acid, and a protein, into a mammalian oocyte.

## Description

### Technical Field

The present invention relates to a promoter for introducing an extracellular substance for a mammalian oocyte containing a tubulin-polymerization inhibitor as an active ingredient, and to a method for introducing an extracellular substance into a mammalian oocyte by using the introducing promoter. More specifically, the present invention relates to a method for introducing an extracellular substance such as a sperm, cell nucleus, nucleic acid, and protein, into a mammalian oocyte by using a promoter for introducing an extracellular substance for a mammalian oocyte containing a tubulin-polymerization inhibitor as an active ingredient, and to a promoter for introducing an extracellular substance for a mammalian oocyte to be used therefor. One characteristic of the invention is to provide a method for promoting fertilization of a mammalian oocyte, by promoting introduction of a sperm into a mammalian oocyte, by enhancing fertility of a mammalian oocyte by a treatment with a promoter for introducing an extracellular substance for a mammalian oocyte containing a tubulin-polymerization inhibitor as an active ingredient.

### Background Art

In the field of mammalian modification using gene therapy, reproductive treatment, or developmental engineering, the introduction of an extracellular substance such as a sperm, cell nucleus, and nucleic acid into a mammalian oocyte is conducted in order to modify germ cells. As a method for introducing genetic (nucleic acid) substances into a cell such as a mammalian oocyte, an introduction method using vectors such as retroviruses is known hitherto (WO97/18318). As a method for introducing an extracellular substance such as a sperm and cell nucleus into a mammalian oocyte, the ICSI (intracytoplasmic sperm injection) method is generally used, in which a hole is made in an oolemma using a glass capillary, thereby infusing an extracellular substance. The ICSI method is also a mainstream method for introducing a sperm into an oocyte of mammalians including human (Japanese Laid-Open Patent Application No.5-38345), and which is broadly used for treatment of human infertility.

However, the ICSI method has some problems as a method for introducing an extracellular substance into a mammalian oocyte. More specifically, the ICSI method has the following problems: (1) technical training is required in order to introduce an extracellular substance into a mammalian oocyte without fail by the ICSI method; (2) the success rate of introducing an extracellular substance into a mammalian oocyte by the ICSI method is low; (3) since the ICSI method is carried out by making a hole in an oolemma using a glass capillary, an adverse effect on embryonic development caused by making a hole can be conceivable. Even though the problems have been pointed out, the ICSI method is in use since there is no alternative method for the ICSI method currently.

On the other hand, introduction of a sperm into a mammalian oocyte is conducted by fertilization, and several methods for promoting introduction of a sperm into a mammalian oocyte are proposed. For example, Published Japanese translation of PCT international publication 8-503705 discloses a method for treating mammalian male infertility by administrating a fertility-enhancing thymosin polypeptide such as thymosin α₁ to promote fertilization. Japanese Laid-Open Patent Application No. 9-28721 discloses a method for improving in vitro fertility of cattle sperm, in which cattle sperm is cocultured with a cattle oviduct epithelial cell, then cultured with a cattle oocyte to undergo in vitro fertilization.

Furthermore, Published Japanese translation of PCT international publication 2001-506225 discloses a method of using angiotensin II to promote fertilization of a mammalian oocyte. Japanese Laid-Open Patent Application No. 6-189650 discloses a method for increasing conception rate by magnetizing a sperm and an oocyte and enhancing the activity thereof. Also, Published Japanese translation of PCT international publication 2001-500102 discloses a method for improving sperm-oocyte binding and enhancing fertility by using a purified polypeptide purified by centrifuging a frozen- and thawed-sperm suspension in the presence of 10% to 12% of glycerol, and being a member of a group of proteins including prosaposin (SGP-1) and saposin. All of the methods are aiming at obtaining an effect of activating an oocyte and sperm, and promoting fertilization thereof during mammalian oocyte fertilization.

On the other hand, in a cell of eukaryotes such as animals, plants, and fungi, it is known that there is an organelle called microtubules, which is scattered in the cell and is tubular protein fibers having a diameter of about 25 nm. The functions of organelles are extremely wide-ranging and involved in mitotic apparatus formation, formation and retention of cell morphology, flagellar/ciliary movement, placement of intracellular organelles, material transportation, hormonal secretion, and cytoplasmic membrane fluidity. Especially, microtubules are present as the main constituent molecules of axons and dendrites in neurons, and contribute to transportation of materials as rails for motor proteins. Microtubules are generally formed by regular polymerization of a heterodimer which consists of one α-tubulin molecule and one β-tubulin molecule, and repeat expanding and contracting caused by polymerization/depolymerization along with cell cycle progression. Moreover, the polymerization/depolymerization of the microtubules is also controlled by Microtubule-Associated Proteins (MAPs, τ protein), and a protein kinase and protein phosphatase containing the microtubule-binding proteins as a main substrate are involved in the control mechanism of microtubules. From these facts, it is known that compounds which act on the microtubule system show various vital activities such as cell division inhibition.

As described above, microtubules are the main components of mitotic spindle fibers which guide chromosomes arranged on the equatorial plate at the time of mitosis to polar points, and play an indispensable roll in many cellular actions including maintenance of the shape, motility, and adhesion of cells, and transportation within cells, during division phase (Shiff, P.B. and Horwitz, S.B., 1981, "Molecular Actions and Targets for Anticancer Chemotherapeutic Agents" section of "Tubulin : a target for chemotherapeutic agents", pp483-507, Academic Press, NY, USA; Glotz, J.S. et al., 1992, Proc. Natl. Acad. Sci., USA, 89:7026-7030; Rowinsky, E.K. et al., 1990, Nature, 82:1247-1259). Also, microtubules play an important role in the interactions between growth factors and receptors on the surface of cells, and in controlling membrane-permeable proliferative signals derived from these interactions. Relationship between fertilization and cytoskeleton has been also reported (Molecular Reproduction and Development, 56:89-98,2000).

Tubulin is the main component protein of microtubules and an indispensable protein for cell division/cell growth, which is a so called cytoskeletal element. Therefore, polymerization inhibition or depolymerization of tubulins has an important relationship with cell division/cell growth. From such functions of tubulin, tubulin is also known as a target of an anticancer agent, an antifungal agent, and herbicides. Also, tubulin has been deeply involved in both the foundation and application in life science.

A number of tubulin-polymerization inhibitors is hitherto known. Vinca alkaloid is a presently well-known tubulin-polymerization inhibitor used to treat cancers (Japanese Laid-Open Patent Application No.5-192174). Vinca alkaloid is a generic term used for referring to one kind of anticancer agents, the component of which is extracted from plants. Vinca alkaloid binds to a protein called tubulin which is involved in cell movement and cell shape maintenance, and inhibits microtubules, whereby inhibiting cell division.

Representative examples of Vinca alkaloid include vinblastine and vincristine (J. Med. Chem. 34:1998,1991). Vinblastine is an indole alkaloid having an antitumor activity among the alkaloids contained in Catharanthus roseus, which acts on microtubules or tubulin which is a component protein of microtubles, to stop cell mitosis in the metaphase stage, thereby inhibiting cell division. Vincristine is now used for treatment of leukemia, malignant lymphoma, pediatric tumor, etc. while vinblastine is used for treatment of non-small cell cancer. In recent years, vinblastine is prepared by a synthesis method (Japanese Laid-Open Patent Application No.2003-64084).

Many other tubulin-polymerization inhibitors such as rhizoxin (Tetrahedron Lett., 34:1035, 1993), combretastatin (Biochemistry, 28:6984, 1989; Japanese Laid-Open Patent Application No.6-207264), 2-styrylquinazolin-4 (3H)-one (SQO)(J. Med. Chem., 33:1721,1990), halistatin 1, 2 (Japanese Laid-Open Patent Applications No.6-279450; No.6-279451), and pironetin or its derivatives (Japanese Laid-Open Patent Application No.11-1434), are known, and the use thereof for antitumor drug and the like has been sought.

Patent Document 1: Japanese Laid-Open Patent Application No. 5-38345
Patent Document 2: Japanese Laid-Open Patent Application No. 5-192174
Patent Document 3: Japanese Laid-Open Patent Application No. 6-189650
Patent Document 4: Japanese Laid-Open Patent Application No. 6-207264
Patent Document 5: Japanese Laid-Open Patent Application No. 6-279450
Patent Document 6: Japanese Laid-Open Patent Application No. 6-279451
Patent Document 7: Japanese Laid-Open Patent Application No. 9-28721
Patent Document 8: Japanese Laid-Open Patent Application No. 11-1434
Patent Document 9: Japanese Laid-Open Patent Application No.2003-64084
Patent Document 10: Published Japanese translation of PCT international publication 8-503705
Patent Document 11: Published Japanese translation of PCT international publication 2001-500102
Patent Document 12: Published Japanese translation of PCT international publication 2001-506225
Patent Document 13: WO97/18318
Non-Patent Document 1: Shiff, P.B. and Horwitz, S.B., 1981, "Tubulin : a target for chemotherapeutic agents" section on "Molecular Actions and Targets for Cancer Chemotherapeutic Agents", pp483-507, Academic Press, NY, USA
Non-Patent Document 2: Glotz, J.S. et al., 1992, Proc. Natl. Acad. Sci., USA, 89:7026-7030
Non-Patent Document 3: Rowinsky, E.K. et al., 1990, Nature, 82:1247-1259
Non-Patent Document 4: Molecular Reproduction and Development, 56:89-98,2000
Non-Patent Document 5: J. Med. Chem. 34:1998,1991
Non-Patent Document 6: Tetrahedron Lett., 34:1035, 1993
Non-Patent Document 7: Biochemistry, 28:6984, 1989
Non-Patent Document 8: J. Med. Chem., 33:1721,1990

### Disclosure of the Invention

### Object to be solved by the present invention

An object of the present invention is to provide a method for introducing an extracelllular substance into a mammalian oocyte, enabling a safe and effective introduction into a mammalian oocyte when introducing an extracellular substance such as a sperm, cell nucleus, nucleic acid, and protein into a mammalian oocyte, which is technically easy. A further object of the present invention is to provide a method for introducing a sperm into a mammalian oocyte, which is technically easy and causes less damage to the mammalian oocyte when introducing a sperm into a mammalian oocyte.

### Means to solve the object

The present inventors have conducted a keen study on a method for introducing an extracellular subustance such as a sperm into a mammalian oocyte, and have found that, by treating a mammalian oocyte with a tubulin-polymerization inhibitor such as vinblastine, a membrane fusion between the oocyte and the sperm is promoted, thus fertilization can be achieved by fusing the sperm and the oocyte without making a hole in an oolemma. The present invention has been thus accomplished.

More specifically, as a conventional method for introducing an extracellular substance such as a sperm into a mammalian oocyte, the ICSI method in which a hole is made in an oolemma with a glass capillary has been used. The ICSI method is technically difficult and the success rate thereof is low. Moreover, there is an adverse effect on embryonic development caused by making a hole in the oolemma with a glass capillary. Therefore, the present inventors have conducted a keen study on a method for introducing a sperm which is technically easy and causes less damages on the oocyte, and focused on development of a promoter for introducing a sperm for a mammalian oocyte, which promotes membrane fusion between an oocyte and a sperm.

Cytoplasmic membrane fusion is indispensable in order that a sperm and oocyte undergo fusion to make a fertilized oocyte. Details regarding a molecular mechanism for controlling membrane fusion during fertilization are unknown, however, it has imerged that microtubules having tubulin as a constituent unit play an important roll from recent studies of inventors. Given this factor, effects of tubulin-polymerization promoter and inhibitor on membrane fusion during fertilization was investigated, and it was found out that tubulin-polymerization inhibitor, vinblastine, has a promotive effect on the sperm fusion to an oolemma. Then, it was found out that this chemical treatment enables sperm to be introduced into an oocyte without making a hole in an oolemma.

That is, in order to investigate a mechanism in which vinblastine treatment promotes membrane fusion, localization of membrane proteins in a mouse oocyte which was fertilized in vitro after vinblastine treatment was examined. The results revealed that localization of a four-transmembrane protein CD9, which is believed to control membrane fusion, was different from that of a wild type. The study hitherto have revealed that it is essential that an area where CD9 is not present on an oolemma is transiently formed by the sperm in order that membrane fusion of fertilization occurs. It has been found that a circular area where CD9 is not present is formed before sperm binding by vinblastine treatment. Furthermore, only one sperm fused to the center of a area where CD9 is not present.

The present invention has been completed on the basis of findings described above, and a method of the present invention can be applied not only to the case of introducing a sperm into a mammalian oocyte, but also to the case of introducing an extracellular substance such as a cell nucleus, nucleic acid, and protein into a mammalian oocyte. That is, the present invention relates to a method for introducing an extracellular substance such as a sperm, cell nucleus, nucleic acid, and protein into a mammalian oocyte by using a promoter for introducing an extracellular substance for a mammalian oocyte containing a tubulin-polymerization inhibitor as an active ingredient, and to an promoter for introducing an extracellular substance for a mammalian oocyte for the same. Furthermore, an aspect of the present invention is to provide a method for promoting fertilization of a mammalian oocyte, by promoting introduction of a sperm into a mammalian oocyte, by enhancing fertility of a mammalian oocyte by a treatment with a promoter for introducing an extracellular substance for a mammalian oocyte containing a tubulin-polymerization inhibitor as an active ingredient.

Specifically, the present invention comprises: (1)a promoter for introducing an extracellular substance for a mammalian oocyte used for introducing an extracellular substance containing a tubulin-polymerization inhibitor as an active ingredient into a mammalian oocyte; (2) the promoter for introducing an extracellular substance for a mammalian oocyte according to (1), wherein the tubulin-polymerization inhibitor is vinblastine; (3) the promoter for introducing an extracellular substance for a mammalian oocyte according to (1) or (2), wherein the introduction of an extracellular substance into a mammalian oocyte is the introduction of a sperm into the mammalian oocyte; (4) the promoter for introducing an extracellular substance for a mammalian oocyte according to (3), wherein the introduction of a sperm into the mammalian oocyte is promoted by enhancement of fertility of the mammalian oocyte; (5) the promoter for introducing an extracellular substance for a mammalian oocyte according to any of (1) to (4), wherein the mammal is a human; and (6) the promoter for introducing an extracellular substance for a mammalian oocyte according to any of (1) to (4), wherein the mammal is selected from the group consisting of a mouse, rat, cattle, pig, horse, sheep, and monkey.

Moreover, the present invention comprises: (7)a method for introducing an extracellular substance into a mammalian oocyte, wherein the mammalian oocyte is treated with a promoter for introducing an extracellular substance for a mammalian oocyte containing a tubulin-polymeraization inhibitor as an active ingredient, when introducing the extracellular substance into the mammalian oocyte; (8) the method for introducing an extracellular substance into a mammalian oocyte according to (7), wherein the mammalian oocyte is treated with vinblastine, when introducing the extracellular substance into the mammalian oocyte; (9) the method for introducing an extracellular substance into a mammalian oocyte according to (7) or (8), wherein the introduction of an extracellular substance into a mammalian oocyte is the introduction of a sperm into a mammalian oocyte; (10) the method for introducing an extracellular substance into a mammalian oocyte according to (9), wherein the introduction of an extracellular substance into a mammalian oocyte is achieved by sperm-oocyte fusion outside a mammalian body; (11) the method for introducing an extracellular substance into a mammalian oocyte according to (9) or (10), wherein the introduction of the sperm into a mammalian oocyte is promoted by improvement of fertility of the mammalian oocyte; and (12) the method for introducing an extracellular substance into a mammalian oocyte according to (11), wherein improvement of the fertility of the mammalian oocyte is promotion of sperm fusion to an oolemma.

### Brief Description of Figures

[Fig. 1]
   It is a figure showing the result of the number of sperm fused with an oocyte as a result of in vitro fertilization of mouse sperm and a zona pellucida-free mouse unfertilized oocyte which has been treated with vinblastine, a tubulin-polymerization inhibitor, in an example of the present invention.
[Fig. 2]
   It is a figure showing the result of the ratio of fertilized oocytes resulted from in vitro fertilization of mouse sperm and an unfertilized mouse oocyte with zona pellucida which has been treated with vinblastine, a tubulin-polymerization inhibitor, in an example of the present invention.

### Best Mode of Carrying Out the Invention

A method of the present invention includes treating the mammalian oocyte with a promoter for introducing an extracellular substance for the mammalian oocyte containing a tubulin-polymerization inhibitor as an active ingredient, and introducing the extracellular substance into the mammalian oocyte when introducing an extracellular substance such as sperm into a mammalian oocyte. As a tubulin-polymerization inhibitor used as an active ingredient of the promoter for introducing an extracellular substance for the mammalian oocyte of the present invention, a tubulin-polymerization inhibitor which is publicly known as an inhibitor for tubulin polymerization, can be used. The tubulin-polymerization inhibitor includes vinca alkaloid, and typical examples of vinca alkaloid include vinblastine and vincristine. As a most preferable active ingredient of the promoter for introducing an extracellular substance for the mammalian oocyte of the present invention, vinblastine can be exemplified.

A mammalian oocyte of interest in the present invention includes, but not especially limited to, an oocyte of a human, mouse, rat, cattle, pig, horse, sheep, and monkey. A method of introducing an extracellular substance into a mammalian oocyte according to the present invention can be applied to extracellular substances such as sperm, a cell nucleus, a nucleic acid, and a protein. However, the method for introducing an extracellular substance according to the present invention can be especially applied to the introduction of a sperm into a mammalian oocyte, thus can be provided as an effective method of fertilization of a mammalian oocyte.

In the present invention, the treatment of a mammalian oocyte with the promoter for introducing an extracellular substance for the mammalian oocyte containing a tubulin-polymerization inhibitor as an active, can be carried out by: collecting an oocyte, culturing it in an adequate medium (e.g. TYH medium) followed by culturing it in the medium added with a tubulin-polymerization inhibitor for a few hours.

Although the invention will be described in more detail in the following by way of examples, the technical scope of the present invention is not limited thereto.

### EXAMPLE 1

### (Increase in fertilization efficiency by vinblastine treatment)

In order to examine the influence of a tubulin-polymerization inhibitor on fertilization of a mouse unfertilized oocyte, vinblastine was used as a tubulin-polymerization inhibitor to treat a mouse unfertilized oocyte. Then the fertility thereof was investigated.

### (Materials and experimental conditions)

### 1. Medium composition (g/L) (TYH medium)

| | |
|---|---|
| NaCl | 6.976 |
| KCl | 0.356 |
| CaCl₂ | 0.190 |
| KH₂PO₄ | 0.162 |
| MgSO₄•7H₂O | 2.930 |
| NaHCO₃ | 2.106 |
| glucose | 1.000 |
| sodium pyruvate | 0.550 |
| penicillin G | 0.750 |
| streptomycin | 0.050 |
| bovine serum albumin F-V | 4.000 |
| 1% phenol red | 1.0 ml |

### 2. acid Tyrode's solution (g/L)

| | |
|---|---|
| NaCl | 8.00 |
| KCl | 0.20 |
| CaCl₂•2H₂O | 0.24 |
| Mg Cl₂•6H₂O | 0.10 |
| Glucose | 1.00 |

Adjust pH to 2.5 with HCl, and filter-sterilized.

### 3. Cultivation temperature: 37°C

### (Operation of experiment)

### 1. Oocyte collection

(1) Mice of eight week-old or older were purchased and made adapted to grow environment for 1 week before use. PMS (Serotoropin exclusive to animal, manufactured by ASUKA Pharmaceutical) was injected in an amount of 0.1 ml into the abdominal cavity of the mouse at 8:00 pm.
(2) 48 hr later, HCG (Gonadotropin exclusive to animal, manufactured by ASUKA Pharmaceutical) was injected in an amount of 0.1 ml into the abdominal cavity.
(3) Mice were euthanized the next day (14 to 16 hr after HCG injection), and the ampulla of oviduct was removed. An oocyte surrounded by cumulus cells was collected and cultured in a (100 µL) drop of TYH medium.
(4) Cumulus cells were removed by hyaluronidase (300 µg/mL) treatment, then the oocyte was cultured in TYH medium for 3 hr. In the case of conducting removal of zona pellucida, oocyte zona pellucida was removed by an acid Tyrode's solution treatment followed by culturing the oocyte in TYH medium for 3 hr.

### 2. Treatment with a tubulin-polymerization inhibitor

The collected unfertilized oocyte of the mice was treated by using vinblastine (VB) as a tubulin-polymerization inhibitor. As controls, an oocyte treated with paclitaxel (Pac; Brand Name: taxol), tubulin-polymerization promoter, and an untreated oocyte (control) were used. Vinblastine (VB) treatment was conducted by culturing the oocyte in 20 µM of vinblastine solution (TYHmedium) for 1 hr, while paclitaxel (Pac) treatment was conducted by culturing the oocyte in 10 µM of paclitaxelel (Pac) solution (TYH medium) similarly for 1 hr.

### 3. In vitro fertilization

The mouse unfertilized oocyte was treated with vinblastine (VB), a tubulin-polymerization inhibitor, and in vitro fertilization was conducted with mouse sperm. As controls, an unfertilized oocyte treated with paclitaxel (Pac; Brand Name: taxol), tubulin-polymerization promoter, and an untreated oocyte (control) were used.

### (Results)

(In the case where zona pellucida-free oocytes were use): To quantify sperm-oocyte fusion efficiency, zona pellucida-free mouse unfertilized oocytes were treated with vinblastine (VB), a tubulin-polymerization inhibitor, or paclitaxel (Pac), tubulin-polymerization promoter, or left untreated(control). Then the oocytes were in vitro fertilized with mouse sperm, respectively. The results are shown in Fig. 1. The figure shows the results of in vitro fertilization (zona pellucida-free oocytes, 1 hr after insemination). In the figure, white bars show the number of fused sperm. When comparing the number of sperm which fused with the oocytes 1 hr after insemination, the VB (vinblastine)-treated oocyte showed the largest number. On the other hand, a large number of sperm stuck to the surface of the paclitaxel (Pac)-treated oocyte but not fused therewith, because the paclitaxel (Pac)-treated oocyte made difficult sperm to be fused.

(In the case where oocytes with zona pellucida are used): Mouse unfertilized oocytes just after ovulation, which had not been undergone special treatment such as removal of zona pellucida, were treated with vinblastine, a tubulin-polymerization inhibitor, and in vitro fertilized with mouse sperm. The results are shown in Fig. 2. An experiment using anti-mouse CD9 antibody having a fusion-inhibitory effect was conducted simultaneously with this experiment. In the case of mouse, about 20% of oocytes cannot be fertilized due to immaturity and over-maturity. However, after investigating the fertilization efficiency using the 2-cell-stage oocytes, the fertilization efficiency of the vinblastine-treated oocytes increased by 20% comparing to that of the untreated oocyte. As it will be described in the followings, even an immature oocyte could be fused with sperm when treated with vinblastine. So, it is believed that this is the result of the increased fusion efficiency of sperm-oocyte fusion due to the treatment with vinblastine. The experimental result reveals that either immature or over-mature oocytes become possible to fuse with sperm by vinblastine treatment. Furthermore, fusion efficiency of oocytes which have been treated with anti-mouse CD9 antibody having a fusion-inhibitory effect, was also increased by vinblastine treatment.

### (Increase of fertilization efficiency by vinblastine treatment)

After treating mouse unfertilized oocytes with vinblastine, a tubulin-polymerization inhibitor, the oocytes were in vitro fertilized with mouse sperm. The results revealed that fertilization efficiency thereof increased by 20% comparing to untreated group. In the case of mouse, about 20% of oocytes cannot be fused with sperm due to immaturity. However, even immature oocytes could fuse with sperm when treated with vinblastine.

### (Treatment with paclitaxelel)

When a mouse unfertilized oocyte was treated with paclitaxelel (Pac), tubulin-polymerization promoter, sperm-oocyte membrane fusion was inhibited. A number of deformed microvilli were observed when the morphology of oocyte membrane was examined with an electron microscope.

### (Formation of sperm fusion area by vinblastine treatment)

The mechanism in which membrane fusion was promoted by vinblastine treatment was investigated. After vinblastine treatment, the localization of membrane proteins in an in vitro fertilized mouse oocyte was examined. When treated with vinblastine, a tubulin-polymerization inhibitor, circular areas where localization of CD9 on the oocyte membrane does not change were formed. The number of circular areas differed depending on oocytes and were ranging from 1 to 6. Moreover, it was confirmed that one sperm was fused to the center of circular areas. This shows that tubulin depolymerization is necessary for sperm fusion.

Namely, the results revealed that the localization of a four-transmembrane protein CD9, which is believed to control membrane fusion, was different from that of a wild type. The study hitherto has revealed that it is essential that an area where CD9 is not present is transiently formed by the sperm on an oolemma for the occurrence of membrane fusion of fertilization. It has been found that a circular area where CD9 is not present is formed by vinblastine treatment before sperm binding. Furthermore, only one sperm fused to the center of an area where CD9 is not present. In addition, it was confirmed that, in fertilization of an oocyte with a semipermeable membrane, the number of sperm to be introduced into the oocyte is limited to one due to its polyspermy block mechanism. From these results, it has became apparent that, by treating the specific area of oocyte membrane with vinblastine, the introduction of sperm into an oocyte becomes possible without using a glass capillary.

### Industrial Applicability

The present invention can provide a method for introducing an extracelllular substance into a mammalian oocyte, enabling a safe and effective introduction into a mammalian oocyte when introducing an extracellular substance such as a sperm, cell nucleus, nucleic acid, and protein into a mammalian oocyte, which is technically easy. Specifically, a method of the present invention can be applied as an introduction method of a sperm into a mammalian oocyte, and provide a technically easy method without using a special device such as that used in the conventional ICSI method, and enables sperm to be introduced into a mammalian oocyte causing less damages on the mammalian oocyte, because it is not necessary to make a hole in the oolemma with a glass capillary as in the ICSI method. Therefore, it can be expected that the present invention can contribute to the development in the field of mammalian modification using gene therapy, reproductive treatment, or developmental engineering, as a useful means for introducing an extracellular substance such as a sperm, cell nucleus, nucleic acid, and protein, in a mammalian oocyte, for fertilization or modification of germ cells.

## Claims

1. A promoter for introducing an extracellular substance for a mammalian oocyte used for introducing an extracellular substance containing a tubulin-polymerization inhibitor as an active ingredient into a mammalian oocyte.

2. The promoter for introducing an extracellular substance for a mammalian oocyte according to claim 1, wherein the tubulin-polymerization inhibitor is vinblastine.

3. The promoter for introducing an extracellular substance for a mammalian oocyte according to claim 1 or 2, wherein the introduction of an extracellular substance into a mammalian oocyte is the introduction of a sperm into the mammalian oocyte.

4. The promoter for introducing an extracellular substance for a mammalian oocyte according to claim 3, wherein the introduction of a sperm into the mammalian oocyte is promoted by enhancement of fertility of the mammalian oocyte.

5. The promoter for introducing an extracellular substance for a mammalian oocyte according to any of claims 1 to 4, wherein the mammal is a human.

6. The promoter for introducing an extracellular substance for a mammalian oocyte according to any of claims 1 to 4, wherein the mammal is selected from the group consisting of a mouse, rat, cattle, pig, horse, sheep, and monkey.

7. A method for introducing an extracellular substance into a mammalian oocyte, wherein the mammalian oocyte is treated with a promoter for introducing an extracellular substance for a mammalian oocyte containing a tubulin-polymeraization inhibitor as an active ingredient, when introducing the extracellular substance into the mammalian oocyte.

8. The method for introducing an extracellular substance into a mammalian oocyte according to claim 7, wherein the mammalian oocyte is treated with vinblastine, when introducing the extracellular substance into the mammalian oocyte.

9. The method for introducing an extracellular substance into a mammalian oocyte according to claim 7 or 8, wherein the introduction of an extracellular substance into a mammalian oocyte is the introduction of a sperm into the mammalian oocyte.

10. The method for introducing an extracellular substance into a mammalian oocyte according to claim 9, wherein the introduction of an extracellular substance into a mammalian oocyte is achieved by sperm-oocyte fusion in vitro.

11. The method for introducing an extracellular substance into a mammalian oocyte according to claim 9 or 10, wherein the introduction of a sperm into a mammalian oocyte is promoted by improvement of fertility of the mammalian oocyte.

12. The method for introducing an extracellular substance into a mammalian oocyte according to claim 11, wherein the improvement of the fertility of the mammalian oocyte is promotion of sperm fusion to an oolemma.
